# EUROPÄISCHE PATENTANMELDUNG

(11) **EP 4 708 309 A1**
(43) Veröffentlichungstag der Anmeldung: **11.03.2026**
(21) Anmeldenummer: 24198644.7
(22) Anmeldetag: 05.09.2024
(51) Int. Cl.: G16H 20/17, A61M 5/142, G16H 40/20, G16H 40/40, G16H 40/63, G16H 40/67, H04L 67/12, H04W 4/80, H04W 84/18

(54) **INFUSIONSPUMPENSYSTEM UND ZUGEHÖRIGES BETRIEBSVERFAHREN**

(71) Anmelder: B. Braun Melsungen AG, 34212 Melsungen (DE)
(72) Erfinder: Schwarz, Jan, 34212 Melsungen (DE); Moeller, Sebastian, 37235 Hessisch Lichtenau (DE); Mauro, Christian, 34560 Fritzlar (DE); Erlen, Christoph, 34132 Kassel (DE); Buerger, Maria, 34323 Malsfeld (DE); Scheler, Stefan, 82110 Germering (DE); Burghardt, Philipp, 81479 München (DE)
(74) Vertreter: Tergau & Walkenhorst Intellectual Property GmbH

(57) **Zusammenfassung**

Hauptanspruch: Verfahren zum Betreiben eines Infusionspumpensystems (6) mit einer Mehrzahl von Infusionspumpen (2), die einem Verbund zugewiesen sind, wobei die jeweilige Infusionspumpe (2) eine Eingabeeinheit (10) zur Eingabe von therapierelevanten Patientendaten und/oder Systemdaten sowie eine Kommunikationsschnittstelle zur Kommunikation mit anderen Infusionspumpen (2) des Verbunds aufweist, wobei an einer der Infusionspumpen (2) eingegebene therapierelevante Patientendaten und/oder Systemdaten automatisch an alle anderen Infusionspumpen (2) des Verbunds übermittelt und dadurch im Verbund synchronisiert werden.

## Beschreibung

Die Erfindung betrifft ein Verfahren zum Betreiben eines Infusionspumpensystems. Sie betrifft ferner ein zur Durchführung des Verfahrens geeignetes Infusionspumpensystem.

EP 2954483 A1 beschreibt ein System zur Verwaltung von Infusionspumpen, bei dem ein Bediener eine medizinische Vorrichtung (z. B. eine Infusionspumpe) mit einer bestimmten Entität wie einem Patienten, einem Ort, anderen medizinischen Geräten oder einem Pflegepersonal assoziieren kann. Diese Assoziation wird durch eine Verwaltungsressource gesteuert, die relevante medizinische Informationen sammelt und speichert. Sobald die Assoziation erfolgt ist, sind die medizinischen Informationen für das Infusionssystem und den Bediener zugänglich, was eine effizientere Verwaltung und Nutzung des Systems ermöglicht.

Die vorliegende Erfindung hat die Aufgabe, ein Infusionspumpensystem und ein zugehöriges Betriebsverfahren zu schaffen, das die Eingabe und Synchronisation von therapierelevanten Patientendaten und Systemdaten zwischen mehreren Infusionspumpen eines Verbunds ermöglicht oder unterstützt, wobei insbesondere Datenintegrität und Datenkonsistenz sichergestellt sein sollen. Das Konzept soll für verschiedene Zuweisungsmethoden anwendbar sein. Dadurch soll ein Patient problemlos von einer Infusionspumpe des Verbunds zur nächsten wechseln können.

Die genannte Aufgabe wird gelöst durch ein Verfahren gemäß Anspruch 1. Das zugehörige Infusionspumpensystem ist in Anspruch 13 definiert. Anspruch 14 spezifiziert eine zur Durchführung des Verfahrens und zur Einbindung in das System geeignete Infusionspumpe.

Demnach lehrt die Erfindung ein Verfahren zum Betreiben eines Infusionspumpensystems mit einer Mehrzahl von Infusionspumpen, die einem Verbund zugewiesen sind, wobei die jeweilige Infusionspumpe eine Eingabeeinheit zur Eingabe von therapierelevanten Patientendaten und/oder Systemdaten sowie eine Kommunikationsschnittstelle zur Kommunikation mit anderen Infusionspumpen des Verbunds aufweist, wobei an einer der Infusionspumpen eingegebene therapierelevante Patientendaten und/oder Systemdaten automatisch an alle anderen Infusionspumpen des Verbunds übermittelt und dadurch im Verbund synchronisiert werden.

Die vorliegende Erfindung betrifft demnach insbesondere ein Verfahren zur Synchronisation von Patientendaten zwischen zwei oder mehr Infusionspumpen, um damit die Eingabe von therapierelevanten Patientendaten an nur einer Infusionspumpe vorzunehmen und den gleichen Patientendatensatz an anderen Infusionspumpen nutzen zu können.

Therapierelevante Patientendaten sind in diesem Kontext vorteilhafterweise: Alter, Größe, Gewicht, Geschlecht, Körperoberfläche und/oder die Gabe von Opioiden. Je nach Betonung oder Gewichtung kann man auch von patientenrelevanten Therapiedaten sprechen.

Für diesen Ansatz ist es zweckmäßig, Kenntnis über die einem Patienten zugeordneten Infusionspumpen zu besitzen. Das heißt, die Zuordnung der Infusionspumpen zu einem Verbund erfolgt bevorzugt patientenorientiert bzw. patientenbasiert.

In einer ersten bevorzugten Variante erfolgt die Zuordnung der einem Patienten zugewiesenen Infusionspumpen durch ein Ordnungssystem (Rack), in das die Infusionspumpen eingesteckt sind. Sie bilden dadurch einen Infusionsarbeitsplatz, der für einen Patienten genutzt wird. Das Rack stellt vorzugsweise eine Kommunikationsplattform zwischen den eingesteckten Infusionspumpen bereit. Eine bevorzugte Lösung umfasst eine optisch/elektrische Kommunikation, die vorteilhafterweise mittels Infrarot-Signal umgesetzt ist. Andere Möglichkeiten über eine kabelgebundene Lösung oder eine Funkverbindung (z. B. Mobilfunk) sind möglich.

In einer zweiten bevorzugten Variante können die Infusionspumpen über eine Serveranwendung einem Patienten zugewiesen und dadurch dem Verbund zugeordnet werden, d. h. die Infusionspumpen werden beispielsweise mit einem eindeutigen Identifikationsmerkmal belegt, das einen Patienten repräsentiert. Für diese zweite Variante ist es erforderlich, dass die Infusionspumpen eine Kommunikationseinheit besitzen, die mit der Serveranwendung bzw. Serverapplikation kommunizieren. Die Kommunikation erfolgt dabei vorzugsweise drahtlos (z. B. WLAN oder Bluetooth), könnte aber auch drahtgebunden stattfinden.

In einer dritten bevorzugten Variante lassen sich die Infusionspumpen über ein Ad-hoc Netzwerk verbinden, d. h. eine Infusionspumpe vernetzt sich mit anderen Infusionspumpen über eine drahtlose Kommunikationsschnittstelle (z. B. WLAN oder Bluetooth) und bildet auf diese Weise einen Verbund von mehreren Infusionspumpen, die zusammen einen Infusionsarbeitsplatz bilden.

Die drei genannten Varianten der Zuweisung (Rack, Server, Ad-hoc) können auch beliebig miteinander kombiniert werden bzw. simultan verwirklicht sein. Beispielsweise kann Ad-hoc Netzwerk bei Ausfall eines Servers imitiert werden, oder ein Server legt in Kombination mit einem Rack den Verbund fest, oder eine erstes Ad-hoc Netzwerk wird über einen Server mit einem zweiten Ad-hoc Netzwerk oder einer einzelnen Infusionspumpe verbunden.

Sobald eine Eingabe an einer Infusionspumpe vorgenommen wird, die zu diesem Verbund gehört, werden die anderen Infusionspumpen zweckmäßigerweise bzgl. ihrer Eingabe von therapierelevanten Patientendaten blockiert. Gleichzeitig werden die therapierelevanten Patientendaten an die anderen Infusionspumpen weitergeleitet, damit diese die neu eingegebenen Daten zur Verfügung haben.

Bei der Übernahme bzw. Übermittlung der Daten sind zweckmäßigerweise zwei Fälle zu unterscheiden: a.) eine Infusionspumpe befindet sich in der Anwendung mit dem Patienten, d. h. sie infundiert ("fördert"), b.) eine Infusionspumpe befindet sich in keiner Anwendung. Sofern der Fall a.) eintritt, werden die therapierelevanten Patientendaten in der (die Eingabe empfangenden) Infusionspumpe zwischengespeichert und ggf. aufgrund von weiteren Eingaben aktualisiert. Sie werden erst dann angewandt, wenn die laufende Therapie abgeschlossen bzw. beendet ist. Mit anderen Worten heißt das, dass sich die zwischengespeicherten Daten aktualisieren, wenn während der Zwischenspeicherung an einer weiteren Infusionspumpe ein Parameter ein weiteres (oder zweites/drittes/viertes/...) Mal aktualisiert wurde. Im Fall b.) werden die therapierelevanten Patientendaten vorzugsweise direkt übernommen und liegen dem Anwender bzw. der (anderen) Infusionspumpe(n) vor.

Mit anderen Worten erfolgt die Anwendung der neu eingegebenen Daten im Fall a) verzögert nach Beendigung der laufenden Infusion und im Fall b) ohne wesentliche Verzögerung und in diesem Sinne "sofort".

In beiden Fällen kann aber vor der Anwendung der Daten eine Verifikation oder Plausibilitätsprüfung vorgesehen sein, bei der die empfangenen Daten mittels Checksumme auf Validität geprüft werden. Zudem werden Zeitstempel und/oder Sequenznummern ausgetauscht, um die eingegangenen Daten auf Plausibilität zu prüfen. Sollten beispielsweise die empfangenen Zeitstempel oder Sequenznummern "veraltet" sein, so werden die empfangenen Daten verworfen.

Es ist generell möglich, die Eingabe der therapierelevanten Patientendaten oder patientenrelevanten Patientendaten zu unterbrechen oder auf einer anderen, weiteren im Verbund befindlichen Infusionspumpe weiterzuführen. Letzteres ist insofern nötig, wenn eine Therapieform weitere patientenrelevante Therapiedaten benötigt, die zuvor nicht eingetragen / vorhanden waren. Auch in diesem Fall gilt vorzugsweise, dass die gleichzeitige Eingabe patientenrelevanter Therapiedaten an anderen, im Verbund befindlichen Infusionspumpen blockiert wird, wobei im Anschluss daran (alternativ direkt, falls keine Blockierung vorliegt) die patientenrelevanten Therapiedaten an die anderen im Verbund befindlichen Infusionspumpen gesendet bzw. übertragen werden.

Des Weiteren lassen sich patientenrelevante Therapiedaten im gesamten Verbund löschen. Dieses Ereignis kann auf einer einzelnen Infusionspumpe ausgelöst werden, wird jedoch zweckmäßigerweise, wie in den oben genannten Fällen a.) und b.) ggf. gestaffelt ausgelöst. Das heißt, eine Infusionspumpe, die eine aktive bzw. laufende Anwendung besitzt, setzt ihre patientenrelevanten Therapiedaten erst nach dem Beenden einer Anwendung zurück, während eine Infusionspumpe, die sich in keiner Anwendung befindet, ihre patientenrelevanten Therapiedaten sofort zurücksetzt.

Generell lässt sich dieses Konzept nicht nur auf den Austausch patientenrelevanter Therapiedaten beschränken, sondern es ist auch möglich, allgemeingültige Daten in einem Verbund auszutauschen und zu synchronisieren. Solche Daten sind beispielsweise die Zuordnung zu einem Bettplatz, die durch einen Nutzer einmalig an einer Pumpe im Verbund bestätigt wird (engl. bedplace assignment / location assignment) oder die Auswahl einer Pflegestation (care unit) in einem Krankenhaus oder einer ähnlichen medizinischen Einrichtung, die ebenfalls im gesamten Verbund verteilt und synchronisiert wird. Weiterhin ist es möglich, einzelnen Settings / Konfigurationsdaten, wie Druckstufeneinstellungen, Sprachauswahlinformation, Helligkeitseinstellungen, Lautstärkeeinstellungen, Uhrzeit- und Datumseinstellungen, das Aktivieren/Deaktivieren eines WLAN-Moduls oder das Aktivieren/Deaktivieren von Barcode-Informationen oder Keep-Vein-Open (KVO) Therapie an einer Infusionspumpe vorzunehmen und im Verbund zu verteilen.

Die Eingabeeinheit muss nicht zwingend Teil der jeweiligen Infusionspumpe sein. Denkbar ist auch eine Eingabe über eine zentrale Eingabeeinheit, ein gekoppeltes Device (etwa Smartphone oder Tablet) oder eine andere Infusionspumpe im Verbund. Die Eingabeeinheit im Sinne der Erfindung ist daher im Allgemeinen eine der Infusionspumpe zugeordnete oder zuordenbare Eingabeeinheit. Insbesondere kann die Eingabeeinheit auch eine dezentrale Eingabeeinheit sein, die nicht ein physischer Bestandteil der Infusionspumpe ist. Beispielsweise eine zentrale oder transportable Eingabeeinheit.

Zusammenfassend betrifft die Erfindung ein Verfahren zur Synchronisation von Patientendaten und/oder Systemdaten zwischen zwei oder mehr Infusionspumpen. Werden an einer Infusionspumpe ein oder mehrere therapierelevante Patientendaten und/oder Systemdaten eingegeben, so werden diese Informationen von der Infusionspumpe, an der die Eingabe erfolgt, an weitere, dem Patienten zugewiesene Infusionspumpen weitergeleitet. Die Zuweisung zu einem Patienten kann durch eine Anordnung in einem Ordnungssystem (Rack) erfolgen oder durch eine Zusammenfassung von Infusionspumpen zu einem gemeinsamen Verbund durch eine Serveranwendung oder ein Ad-hoc Netzwerk.

Das ist Bezug auf das Verfahren Gesagte überträgt sich analog auf das Infusionspumpensystem und umgekehrt.

Verschiedene Ausführungsbeispiele der Erfindung werden anhand der beigefügten Zeichnungen näher erläutert. Es zeigt:
- FIG. 1: einen Verbund von Infusionspumpen in einem Rack,
- FIG. 2: einen Verbund von Infusionspumpen in einer Client-Server Architektur,
- FIG. 3: einen Verbund von Infusionspumpen in einem Ad-hoc Netzwerk, und
- FIG. 4 - 11: verschiedene Screenshots einer grafischen Benutzerschnittstelle einer einem Verbund zugeordneten Infusionspumpe.

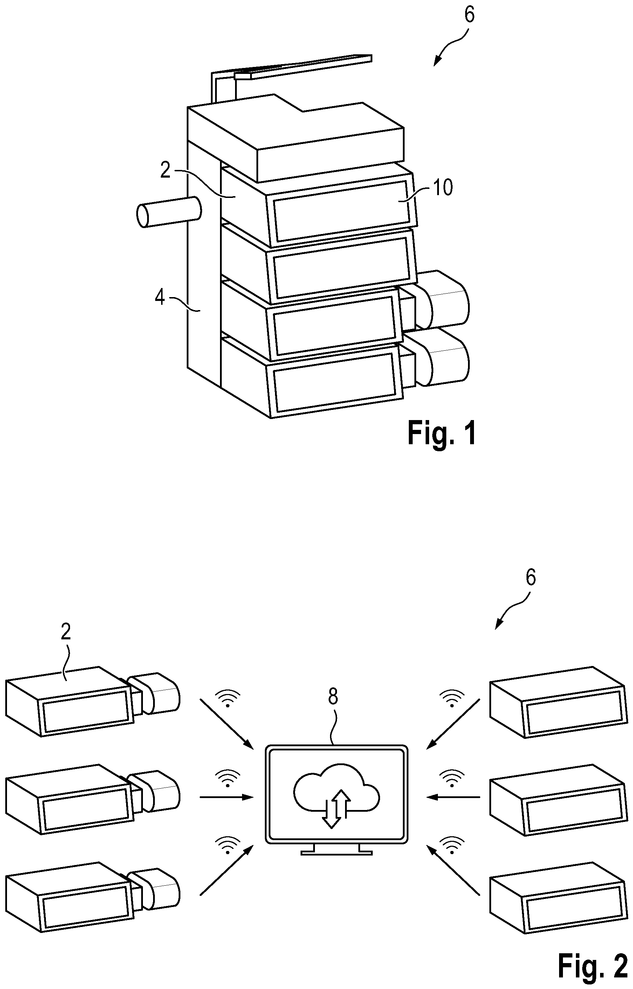

FIG. 1 zeigt eine perspektivische Ansicht einer Mehrzahl von unterschiedlichen medizinischen Infusionspumpen 2, die in ein gemeinsames Rack 4 eingesteckt und dadurch zu einem Verbund (Infusionspumpensystem 6) zusammengeschlossen sind. Die Infusionspumpen 2 weisen jeweils Kommunikationsschnittstellen auf, durch die sie innerhalb des Racks 4 miteinander kommunizieren können. Die Datenübertragung erfolgt über in das Rack 4 integrierten Datenleitungen, optische Übertrager (Infrarot) und Steckanschlüsse.

Ein alternativer Verbund von miteinander kommunizierenden Infusionspumpen 2 ist in FIG. 2 schematisch dargestellt. Die Infusionspumpen 2 bilden dabei Endgeräte eines Client-Server Netzwerkes mit einem zentralen Server 8, der die Zuordnung der Infusionspumpen 2 zum Verbund und die Kommunikation untereinander steuert (die Netzwerktopologie muss allerdings nicht zwingend sternförmig sein).

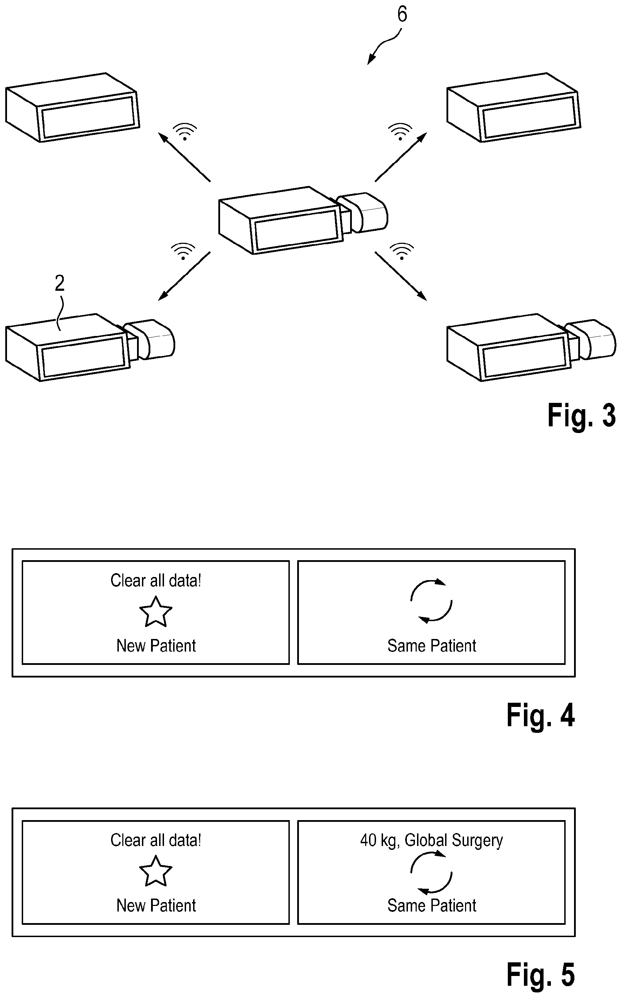

In der Variante gemäß FIG. 3 sind mehrere Infusionspumpen 2 nach Art eines Ad-hoc Netzwerks zu einem Verbund kommunizierend miteinander zusammengefasst. Ein Ad-hoc Netzwerk ist ein dezentrales Netzwerk, in dem die beteiligten Geräte direkt und ohne zentrale Infrastruktur oder festgelegte Router miteinander kommunizieren (Peer-to-Peer Kommunikation). Die Geräte erkennen und verbinden sich automatisch miteinander. Durch eine dynamische Topologie können sich die Geräte im Prinzip frei bewegen und das Netzwerk kann sich entsprechend anpassen.

Erfindungsgemäß ist in jedem der drei Szenarien eine automatische Übertragung oder Übermittlung oder Weiterleitung (vorzugsweise mittels "Push" oder durch Broadcast oder alternativ durch Unicast) von therapierelevanten Patientendaten und/oder Systemdaten, die an einer der Infusionspumpen 2 über eine Eingabeeinheit 10 eingegeben werden, an die anderen Infusionspumpen 2 des Verbundes vorgesehen, so dass eine systemweite Verteilung und Synchronisation dieser Daten stattfindet. Durch die automatische Synchronisation der Daten wird sichergestellt, dass alle Infusionspumpen 2 des Verbunds stets aktuelle therapierelevante Informationen besitzen, was die Effizienz und Sicherheit der Therapie verbessert.

Dabei wird vorteilhafterweise durch Sperrung anderer Infusionspumpen 2 während der Eingabe und Übermittlung von Daten, sowie durch Zwischenspeicherung und spätere Übertragung bei laufenden Infusionen die Integrität und Konsistenz der synchronisierten Daten gewährleistet.

Der Prozess der Dateneingabe und -weiterleitung wird rein exemplarisch anhand von FIG. 4 - 11 veranschaulicht, die verschiedene Screenshots einer grafischen Benutzeroberfläche (z. B. Touch-Screen als Verwirklichung einer Eingabeeinheit 10) einer für die Dateneingabe ausgewählten Infusionspumpe 2 zeigen.

In FIG. 4 tippt der Benutzer im Menu auf "New Patient" (neuer Patient), wodurch die Dateneingabe gestartet und zugleich die Dateneingabe auf den anderen Infusionspumpen 2 des Verbunds blockiert wird. FIG. 5 zeigt den Fall eines bereits erfassten Patienten mit bereits hinterlegten, in den entsprechenden Eingabemasken hinterlegten bzw. vor-ausgefüllten Default-Werten.

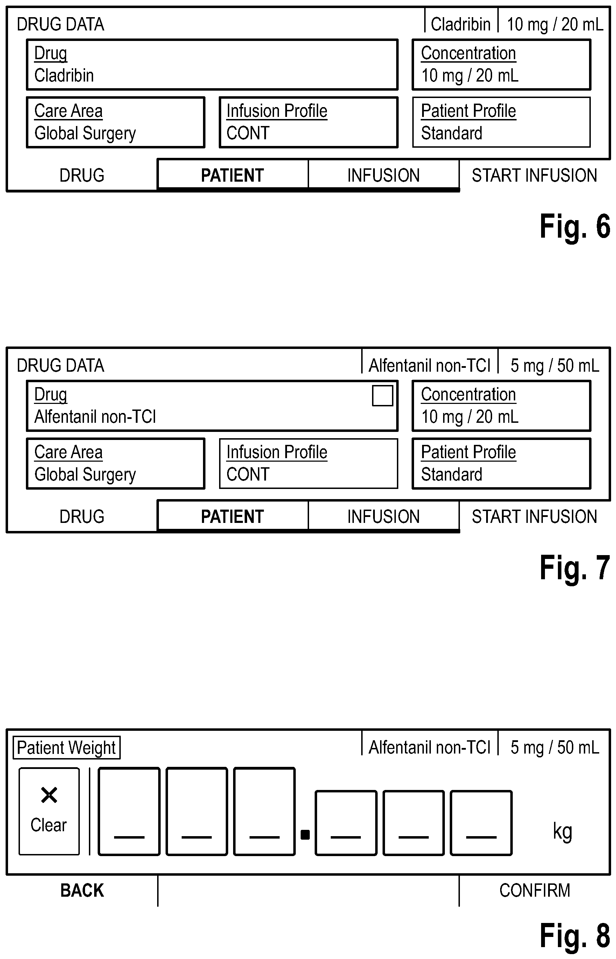

Im folgenden Dialog wird gemäß FIG. 6 das zu verabreichende Medikament bzw. die Infusionslösung (hier: Cladribin) aus einer Auswahlliste gewählt, die auch Angaben zur Konzentration und zum (zeitlichen) Infusionsprofil enthält (hier: kontinuierlich). FIG. 7 zeigt eine mögliche alternative Auswahl (hier: Alfentanil).

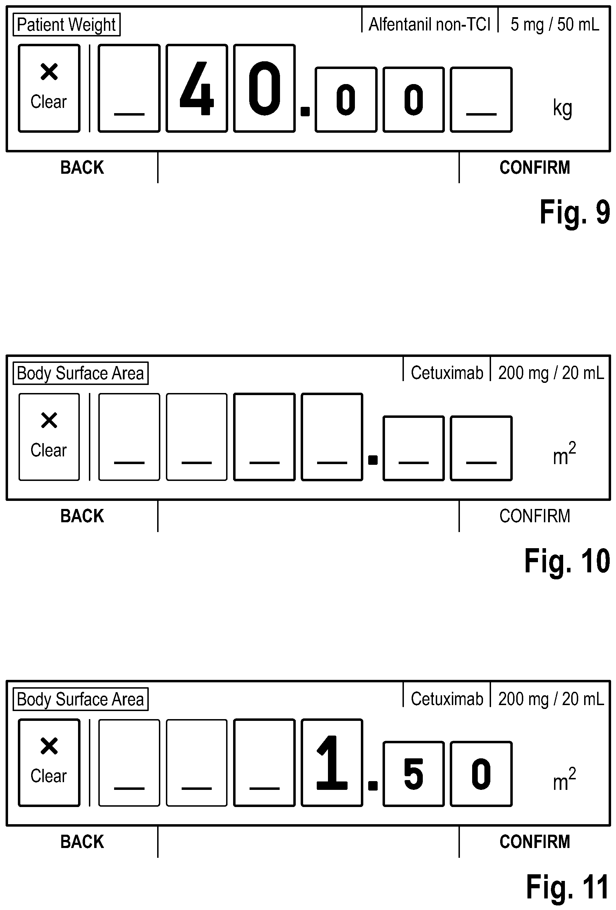

Durch Tippen auf "Patient" gelangt der Benutzer zu einer in FIG. 8 und 9 dargestellten Eingabemaske, in der zum ausgewählten Medikament erforderliche, therapierelevante Patientendaten abgefragt werden (hier für das Medikament Alfentanil: Gewicht des Patienten in kg). FIG. 8 zeigt die noch leere Eingabemaske, FIG. 9 die vom Benutzer ausgefüllte Eingabemaske.

Durch Tippen auf "Confirm" (Bestätigen) wird der eingegebene Wert übernommen. Der eigegebene Wert wird dann an die anderen Infusionspumpen 2 des Verbunds übertragen und damit systemweit synchronisiert. Dadurch wird dieser Wert bei einer nachfolgenden Infusion angewendet, ganz gleich an welcher Infusionspumpe 2 des Verbunds sie letztlich stattfindet. Nach erfolgter Synchronisation wird die Eingabe an den anderen Infusionspumpen 2 wieder entsperrt.

FIG. 10 und 11 zeigen eine alternative Abfrage und Eingabe (hier für das Medikament Cetuximab: Köperfläche im m²).

Selbstverständlich kann ein zu erfassender und zu übermittelnder Patientendatensatz auch mehr als eine Abfrage / Eingabe umfassen oder jede Kombination davon.

Falls die Infusionspumpe, an der die Patientendaten eingegeben werden, während der Eingabe sich in einem aktiven Infusionsmodus befindet, wird die Eingabe dort zwischengespeichert und ggf. anhand von weiteren Eingaben aktualisiert.

Das heißt in einer bevorzugten Implementierung: Wird an einer bereits aktiven bzw. "fördernden" Infusionspumpe ein Parametersatz geändert, so wird dieser stets an alle anderen Infusionspumpen übertragen (und auch an der Pumpe, an der die Eingabe erfolgt, übernommen). Die anderen Infusionspumpen übernehmen diesen Wert umgehend, sofern keine aktive Therapie (fördern) besteht, anderenfalls erfolgt die Aktualisierung nach Beendigung der Therapie.

Das Löschen eines Patientendatensatzes oder eines oder mehrerer Werte daraus wird im Prinzip wie eine Eingabe behandelt und wie oben beschrieben durchgeführt.

### Bezugszeichenliste

- 2: Infusionspumpe
- 4: Rack
- 6: Infusionspumpensystem
- 8: Server
- 10: Eingabeeinheit

## Patentansprüche

1. Verfahren zum Betreiben eines Infusionspumpensystems (6) mit einer Mehrzahl von Infusionspumpen (2), die einem Verbund zugewiesen sind, wobei die jeweilige Infusionspumpe (2) eine Eingabeeinheit (10) zur Eingabe von therapierelevanten Patientendaten und/oder Systemdaten sowie eine Kommunikationsschnittstelle zur Kommunikation mit anderen Infusionspumpen (2) des Verbunds aufweist, wobei an einer der Infusionspumpen (2) eingegebene therapierelevante Patientendaten und/oder Systemdaten automatisch an alle anderen Infusionspumpen (2) des Verbunds übermittelt und dadurch im Verbund synchronisiert werden.

2. Verfahren nach Anspruch 1, wobei eine Zuweisung von Infusionspumpen (2) zum Verbund durch ein Rack (4) erfolgt, in das die Infusionspumpen (2) eingesteckt werden.

3. Verfahren nach Anspruch 2, wobei die Kommunikationsschnittstelle in das Rack (2) integriert ist.

4. Verfahren nach einem der vorangehenden Ansprüche, wobei eine Zuweisung von Infusionspumpen (2) zum Verbund durch einen Server (8) erfolgt, der die Infusionspumpen (2) verwaltet und ihnen bevorzugt spezifische Patienten zuordnet.

5. Verfahren nach einem der vorangehenden Ansprüche, wobei eine Zuweisung von Infusionspumpen (2) zum Verbund durch ein Ad-hoc Netzwerk erfolgt, das durch eine der Infusionspumpen (2) initiiert wird.

6. Verfahren nach einem der vorangehenden Ansprüche, wobei die therapierelevanten Patientendaten Alter, Größe, Gewicht, Geschlecht, Körperoberfläche eines Patienten und/oder Gabe von Opioiden umfassen.

7. Verfahren nach einem der vorangehenden Ansprüche, wobei die Systemdaten Konfigurationseinstellungen wie Druckstufeneinstellungen, Sprachauswahlinformation, Helligkeitseinstellungen, Lautstärkeeinstellungen, Uhrzeit- und Datumseinstellungen, das Aktivieren/Deaktivieren eines WLAN-Moduls, das Aktivieren/Deaktivieren von Barcode-Informationen, und/oder das Aktivieren/Deaktivieren einer Keep-Vein-Open (KVO) Therapie umfassen.

8. Verfahren nach einem der vorangehenden Ansprüche, wobei die Systemdaten die Zuordnung einer Infusionspumpe (2) zu einem Bettplatz und/oder die Auswahl einer Pflegestation innerhalb eines Krankenhauses umfassen.

9. Verfahren nach einem der vorangehenden Ansprüche, wobei während der Eingabe und Übermittlung von therapierelevanten Patientendaten und/oder Systemdaten an/von einer der Infusionspumpen (2) alle anderen Infusionspumpen (2) des Verbunds für die Eingabe von therapierelevanten Patientendaten und/oder Systemdaten gesperrt werden.

10. Verfahren nach einem der vorangehenden Ansprüche, wobei während einer laufenden Infusion an einer der Infusionspumpen (2) eingegebene therapierelevante Patientendaten und/oder Systemdaten dort zwischengespeichert und erst nach Beendigung der Infusion angewendet werden.

11. Verfahren nach einem der vorangehenden Ansprüche, wobei außerhalb einer laufenden Infusion die Übermittlung sofort nach Eingabe der therapierelevanten Patientendaten erfolgt.

12. Verfahren nach einem der vorangehenden Ansprüche, wobei nach entsprechender Benutzereingabe patientenrelevante Therapiedaten im gesamten Verbund gelöscht werden, und wobei eine Infusionspumpe (2), die sich in einer laufende Anwendung befindet, ihre patientenrelevanten Therapiedaten erst nach dem Beenden der Anwendung zurücksetzt, während eine Infusionspumpe (2), die sich in keiner Anwendung befindet, ihre patientenrelevanten Therapiedaten sofort zurücksetzt.

13. Infusionspumpensystem (6) mit einer Mehrzahl von Infusionspumpen (2), die einem Verbund zugewiesen sind, wobei die jeweilige Infusionspumpe (2) eine Eingabeeinheit (10) zur Eingabe von therapierelevanten Patienten und/oder Systemdaten sowie eine Kommunikationsschnittstelle zur Kommunikation mit anderen Infusionspumpen (2) des Verbunds aufweist, wobei die jeweilige Infusionspumpe eine Steuerung aufweist, die an der Eingabeeinheit (10) eingegebene therapierelevante Patientendaten und/oder Systemdaten automatisch an alle anderen Infusionspumpen (2) des Verbunds übermittelt und dadurch im Verbund synchronisiert.

14. Infusionspumpe (2), die eine Eingabeeinheit (10) zur Eingabe von therapierelevanten Patientendaten und/oder Systemdaten sowie eine Kommunikationsschnittstelle zur Kommunikation mit anderen Infusionspumpen (2) eines Verbunds von Infusionspumpen (2) aufweist, wobei die Infusionspumpe eine Steuerung aufweist, die an der Eingabeeinheit (10) eingegebene therapierelevante Patientendaten und/oder Systemdaten automatisch an alle anderen Infusionspumpen (2) des Verbunds übermittelt und dadurch im Verbund synchronisiert.
